# EUROPÄISCHE PATENTANMELDUNG

(11) **EP 0 750 884 A1**
(43) Veröffentlichungstag der Anmeldung: **02.01.1997**
(21) Anmeldenummer: 96110297.7
(22) Anmeldetag: 26.06.1996
(51) Int. Cl.: A61B 10/00

(54) **Verwendung eines Klebefilmes**

(30) Priorität: 29.06.1995 DE 19523581
(71) Anmelder: Beiersdorf Aktiengesellschaft, D-20253 Hamburg (DE)
(72) Erfinder: Ihln, Wernfried, 20257 Hamburg (DE); Khazaka, Gabriel, 50859 Köln (DE)

(57) **Zusammenfassung**

Gegenstand der Erfindung ist die Verwendung eines gefärbten oder farbigen, durchsichtigen oder durchscheinenden Klebefilmes zum Aufnehmen von Proben wie z.B. Zellen von Körperoberflächen, insbesondere von Hautproben von der Hautoberfläche

## Beschreibung

Die Erfindung betrifft die Verwendung eines Klebefilmes für die Probenahme und für die meßtechnische Auswertung der Probe.

Es ist bereits aus der US-Patentschrift 3,965,888 bekannt, zum Sammeln von Körperproben durchsichtige, klebende Kunststoffhalter zu verwenden, die es gestatten, die Probe unter dem Mikroskop zu betrachten.

Weiterhin ist aus der US-Patentschrift 5,088,502 eine Vorrichtung für Hautproben bekannt, die aus einem undurchsichtigen Träger mit einem dunkelen, lichtabsorbierenden Aufdruck besteht, der einen durchsichtigen Klebstoff trägt, auf dem Hautzellen haften, die dann mit bloßem Auge vor dem dunkelen Hintergrund, der den Kontrast erhöht, betrachtet werden können. Diese Vorrichtung ist nicht lichtdurchlässig.

Schließlich ist aus der französischen Patentschrift 2 599 500 ein starres, durchsichtiges Plättchen bekannt, das zur Diagnose von Parasitenproben bestimmt ist und eine Selbstklebemasse trägt, die zusätzlich ein aktives, z.B. chemisches oder färbendes Mittel enthalten kann, das zur Behandlung der Proben dient.

Diese bekannten Vorrichtungen für die Probenahme und deren Verwendungsmöglichkeiten sind jedoch nicht immer befriedigend, insbesondere im Hinblick auf die Bewertung des Probenmaterials durch optische, elektronische Meßverfahren. Zur Ermittlung der Anzahl der Hautoberschichten, die auf dem Klebematerial haften, wird schon eine Einfärbung der Hautschichten durchgeführt. Diese ist zeitaufwendig und mit Fehlern behaftet. Aufgabe der Erfindung ist es daher, ohne eine nachhaltige Einfärbung der Hautproben, eine bessere, einfache Vorrichtung und Anwendung zu schaffen, die diese Nachteile nicht beinhalten.

Aufgabe der Erfindung ist es daher, bessere Vorrichtungen und Anwendungen zu schaffen, die diese Nachteile nicht besitzen.

Diese Aufgabe wird gelöst durch die Verwendung eines gefärbten oder farbigen, durchsichtigen oder durchscheinenden Klebefilmes zum Aufnehmen von Proben wie z.B. Zellen von Körperoberflächen, insbesondere von Hautproben von der Hautoberfläche.

Insbesondere kann dieser, mit dem Probematerial versehene Klebefilm, in einem Durchlichtverfahren verwendet werden und z.B. meßtechnisch ausgewertet werden.

Die Verwendung eines Durchlichtmeßverfahrens zur Auswertung ist sehr vorteilhaft, weil es gestattet, die Dicke bzw. Stärke von Zellen, beispielsweise Hautzellen der Hautproben, insbesondere von Corneozyten objektiv festzustellen.

Auf diese Weise gelingt es, die Messung nicht nur zur Erfassung der Hautprobe nach Flächen durchzuführen, sondern auch die Dicke der anhaftenden Hautprobe zu erfassen. Dies ist z.B. für die Beurteilung der Trockenheit der Hornschicht oder z.B. solcher Hautzellen oder auch anderer Zellen von großer Bedeutung. Insbesondere läßt sich so die Anzahl der Hautoberschichten, die auf dem Klebematerial haften, ermitteln.

Schließlich ist auch die auf dem Klebefilm haftende Hautprobe ohne weitere Hilfsmittel, wie z.B. einem dunklen Untergrund, gut erkennbar und für die Messungen gut auszuwählen.

Der erfindungsgemäße gefärbte oder farbige Klebefilm besteht aus einem streifenförmigen, blattförmigen oder bahnförmigen, vorzugsweise flexiblen Träger, der auf einer Seite mit einer Selbstklebemasse, d.h. druckempfindlichen Klebemasse versehen ist und zum Schutz der freien Oberfläche der Selbstklebemasse eine Schutzabdeckung tragen kann, z.B. wenn er nicht als Rolle aufgewickelt ist. Zu Rollen aufgewickelte Klebefilmbänder können eine antiadhäsive Beschichtung auf der nichtklebenden Rückseite tragen.

Die Färbung des Klebefilms kann durch eine Einfärbung des Trägermaterials und/oder der Selbstklebemasse erreicht werden, aber auch durch einen Farbauftrag oder Farbaufdruck auf dem Träger, z.B. auf der klebmassebeschichteten Seite oder der freien Rückenseite oder auf beiden Seiten. Vorzugsweise ist das Trägermaterial gefärbt.

Besonders bevorzugt wird die Farbe Rot. Der Klebefilm kann aber auch in allen anderen Farbtönen gefärbt sein. Zu berücksichtigen ist, daß der Klebefilm nur in solcher Stärke gefärbt ist, daß er ausreichend durchsichtig oder durchscheinend ist, insbesondere für sichtbares Licht. Die Lichtstärke der Meßgeräte ist dem Fachmann bekannt, so daß eine Abstimmung mit der Stärke der Färbung ohne weiteres möglich ist, wenn unterschiedliche Farben oder Farbtöne verwendet werden.

Als Farben können die bekannten anorganischen oder organischen Farbmittel dienen, die löslich oder unlöslich (Pigmente) sein können.

Zweckmäßigerweise werden Farben und Farbmengen auch so gewählt, daß die gewonnenen Proben nicht verfärbt werden können, z.B. bei längerer Lagerung. Die erfindungsgemäßen Klebefilme oder die Farbe wirkt nicht als Färbemittel oder aktiv auf das Probematerial. Auch aus diesem Grund werden farbige Träger bevorzugt.

Der Gewichtsanteil der Farbstoffe oder Pigmente in dem Klebefilm oder dem Träger kann z.B. 0,01 bis 5 Gew.-%, insbesondere 0,1 bis 1 Gew.-% betragen.

Vorzugsweise kann die Lichtdurchlässigkeit des farbigen Klebefilmes im Bereich bis zu 70%, bevorzugt bis zu 50%, insbesondere aber bis zu 30% unter der Lichtdurchlässigkeit des entsprechenden farblosen Klebefilmes liegen.

Farbige Klebefilme sind an sich bekannt. Eine Vielzahl von Materialien wird für Träger oder Selbstklebemassen verwendet. Bevorzugt werden durchsichtige oder durchscheinende Träger oder Selbstklebemassen.

Als Trägermaterial wird vorzugsweise ein Polymerisat auf Polyolefin-Basis, z.B. Polyäthylen, Polypropylen oder ein Copolymerisat davon oder auch Polyvinylchlorid verwendet. Die Dicke des Trägers kann z.B. 20 - 200 µm, vorzugsweise 50 - 100 µm betragen.

Als Trägerfilme oder -Folien können auch Laminate verwendet werden.

Als Selbstklebemasse können die bekannten Selbstklebemassen oder Haftkleber verwendet werden, z.B. solche auf der Basis von Polyacrylaten oder bevorzugt Natur-Kautschuk und Synthese-Kautschuk, die in bekannter Weise Zusätze von Harzen und Weichmachern enthalten können.

Gut geeignet ist ein unter dem Handelsnamen 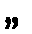 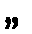 tesafilm 4235 (rot)" ^{R} oder 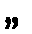 tesafilm 4963 (rot)"^{R} (Fa. Beiersdorf, Hamburg, DE) bekannter Klebefilm.

Bevorzugt werden menschliche und tierische Hautoberflächen für die erfindungsgemäßen Verwendungen ausgewählt. Zur Anwendung wird der Klebefilm mit der Klebefläche auf die zu untersuchende Oberfläche gedrückt oder geklebt und mit anhaftendem Probematerial abgezogen und anschließend direkt vermessen oder mit einer Schutzabdeckung, z.B. üblichen Abdeckfolien wie Klarsichtfolien oder Papieren, die gegebenenfalls mit einer Release - Beschichtung versehen sind, abgedeckt und bis zur Messung aufbewahrt.

Alle Mengenangaben oder Prozentangaben oder Teile beziehen sich, soweit nicht anders angegeben, auf das Gewicht, insbesondere auf das Gesamtgewicht der Zubereitungen oder der jeweiligen Mischung.

## Patentansprüche

1. Verwendung eines gefärbten oder farbigen, durchsichtigen oder durchscheinenden Klebefilmes zum Aufnehmen von Proben wie z.B. Zellen von Körperoberflächen, insbesondere von Hautproben von der Hautoberfläche.

2. Verwendung gemäß Anspruch 1, dadurch gekennzeichnet, daß der Klebefilm rot gefärbt ist.

3. Verwendung eines gefärbten oder farbigen, durchsichtigen oder durchscheinenden Klebefilmes zum Aufnehmen von Proben wie z.B. Zellen von Körperoberflächen, insbesondere von Hautproben von der Hautoberfläche und nachfolgende Verwendung des mit dem Probematerial versehenen Klebefilmes in einem Durchlichtverfahren.

4. Verwendung gemäß Anspruch 1, dadurch gekennzeichnet, daß der Klebefilm-Träger aus einem Polymerisat auf Polyolefin-Basis besteht und als Selbstklebemassen des Klebefilmes solche auf der Basis von Kautschuk verwendet werden.
